# EUROPEAN PATENT APPLICATION

(11) **EP 1 712 622 A1**
(43) Date of publication of application: **18.10.2006**
(21) Application number: 04807956.0
(22) Date of filing: 28.12.2004
(51) Int. Cl.: C12N 15/10, C12Q 1/68, G01N 33/543

(54) **METHOD OF DETECTING TARGET SUBSTANCE**

(30) Priority: 29.12.2003 JP 2003437000
(71) Applicant: Universal Bio Research Co., Ltd., Matsudo-shi, Chiba 271-0064 (JP)
(72) Inventor: TAJIMA, Hideji, c/o UNIVERSAL BIO RESEARCH CO. LTD, Matsudo-shi, Chiba 2710064 (JP)
(74) Representative: Bohnenberger, Johannes
(86) International application number: PCT/JP2004/019601
(87) International publication number: WO 2005/063982

(57) **Abstract**

In order to separate a complex of a first probe, a second probe and a target substance from a reaction mixture obtained by reacting the first probe having first particle (such as labeled particle) and a first binding substance (such as a first oligonucleotide) immobilized on the first particle, the second probe having second particle (such as magnetic particle) and a second binding substance (such as a second oligonucleotide) immobilized on the second particle, and the target substance (such as a target nucleic acid) under mild conditions which do not cause destruction or degradation of the complex, the reaction mixture is mixed with a liquid having a specific gravity greater than that of the first particle, less than that of the second particle and less than that of a particle complex composed of the first particle and the second particle, or a liquid having a specific gravity less than that of the first particle, greater than that of the second particle and greater than that of a particle complex composed of the first particle and the second particle, and then allowed to stand undisturbed until a precipitate and a floating substance are formed.

## Description

### TECHNICAL FIELD

The present invention relates to a method for separating a complex consisting of a first probe, a second probe and a target substance from a reaction mixture obtained by reacting a first probe, a second probe and a target substance, a method for reacting a first probe, a second probe and a target substance, and a method for detecting a target substance using a first probe and a second probe.

### BACKGROUND ART

A known example of a method for detecting a target nucleic acid consists of using a detecting probe, consisting of a first oligonucleotide, which hybridizes with a first segment of a target nucleic acid, and a label added to the first oligonucleotide, and a capturing probe, consisting of a second oligonucleotide, which hybridizes with a second segment of a target nucleic acid, and a capturing group added to the second oligonucleotide (Japanese Patent Application Laid-open No. H6-311899). Inthismethod, after forming hybrids of the detecting probe and capturing probe with the target nucleic acid, the hybrids are separated using the capturing group of the capturing probe, and the hybrid is detected based on the label of the detecting probe.

In the above-mentioned method, if a labeled particle is used for the label added to the first polynucleotide, label intensity can be enhanced and detection accuracy for the target nucleic acid can be improved. Thus, the target nucleic acid can be detected even if there is only a small amount of target nucleic acid (namely, without having to amplify the target nucleic acid by PCR and so on).

In addition, in the above-mentioned method, if a magnetic particle is used for the capturing group added to the second oligonucleotide, the formation and separation of the above-mentioned hybrids can be carried out easily and rapidly by controlling magnetic force. An example of a known method for separating magnetic particles by controlling magnetic force consists of using a dispenser (Japanese Patent No. 3115501 and Japanese Patent Application Laid-open No. H8-320274). In this method, a magnet is arranged in a pipette tip of a disperser which aspirates and discharges a liquid from a container, and although the magnetic particles in the liquid aspirated into the pipette tip are retained on the inner walls of the pipette tip by the action of the magnet, the magnetic particles are released from the inner walls of the pipette tip and discharged to the outside along with the liquid as a result of being no longer subjected to the action of the magnet.

### DISCLOSURE OF THE INVENTION

In the above-mentioned method, in the case of using a first probe having a labeled particle and a first oligonucleotide immobilized on the labeled particle, and a second probe having a magnetic particle and a second oligonucleotide immobilized on the magnetic particle, a hybrid of the first probe, second probe and a target nucleic acid becomes a giant molecule containing the labeled particle and the magnetic particle. Thus, if the above-mentioned formation and separation of the hybrid is carried out using a dispenser, there is the risk of the above-mentioned hybrid being destroyed or degraded by the shock during aspiration and discharge of the dispenser.

Therefore, an object of the present invention is to provide a method for separating a complex from a reaction mixture obtained by reacting a first probe having a first particle (for example, labeled particle) and a first binding substance (for example, a first oligonucleotide) immobilized on the first particle, a second probe having a second particle (for example, magnetic particle) and a second binding substance (for example, a second oligonucleotide) immobilized on the second particle, and a target substance (for example, a target nucleic acid), under mild conditions which do not cause destruction or degradation of the complex of the first probe, second probe and target substance.

In addition, an object of the present invention is to provide a method for reacting a first probe having a first particle (for example, labeled particle) and a first binding substance (forexample, a first oligonucleotide) immobilized on the first particle, a second probe having a second particle (for example, magnetic particle) and a second binding substance (for example, a second oligonucleotide) immobilized on the second particle, and a target substance (forexample, a target nucleic acid), under mild conditions which do not cause destruction or degradation of a complex of the first probe, second probe and target substance.

Moreover, an object of the present invention is to provide a method for detecting a target substance (for example, a target nucleic acid) using a first probe having a first particle (for example, labeled particle) and a first binding substance (for example, a first oligonucleotide) immobilized on the first particle, and a second probe having a second particle (for example, magnetic particle) and a second binding substance (for example, a second oligonucleotide) immobilized on the second particle, under mild conditions which do not cause destruction or degradation of a complex of the first probe, the second probe and the target substance.

In order to solve the above-mentioned problems, a first invention provides a method for separating a complex of a first probe, a second probe and a target substance from a reaction mixture obtained by reacting the first probe having a first particle and a first binding substance immobilized on the first particle, the second probe having a second particle having a different specific gravity from the first particle and a second binding substance immobilized on the second particle, and the target substance having a first portion able to be bound by the first binding substance and a second portion able to be bound by the second binding substance, the method comprising a step in which, after mixing the reaction mixture with a liquid having a specific gravity greater than that of the first particle, less than that of the second particle and less than that of a particle complex composed of the first particle and the second particle, or a liquid having a specific gravity less than that of the first particle, greater than that of the second particle and greater than that of a particle complex composed of the first particle and the second particle, the mixture is allowed to stand undisturbed until a precipitate and a floating substance are formed.

A reaction mixture obtained by reacting a first probe, a second probe and a target substance can include unreacted first probe (first probe not bound to the target substance), unreacted second probe (second probe not bound to the target substance), unreacted target substance (target substance not bound to either the first probe or the second probe), a complex of the first probe and the target substance, a complex of the second probe and the target substance, and a complex of the first probe, the second probe and the target substance. However, all of these are not necessarily required to be contained in the reaction mixture. For example, a complex of the first probe and target substance or a complex of the second probe and the target substance may not be contained depending on the reaction procedure, reaction conditions and so on.

Since the mass and volume of the first particle are much greater than the mass and volume of the first binding substance, the mass and volume of the second particle are much greater than the mass and volume of the second binding substance, and the mass and volume of the first and second particles are much greater than the mass and volume of the target substance, the specific gravity of the first probe approximates the specific gravity of the first particle, the specific gravity of the second probe approximates the second particle, the specific gravity of a complex of the first probe and the target substance approximates the specific gravity of the first particle, the specific gravity of a complex of the second probe and the target substance approximates the specific gravity of the second particle, and the specific gravity of a complex of the first probe, the second probe and the target substance approximates the specific gravity of a particle complex composed of the first particle and the second particle.

Thus, in the case the specific gravity of the second particle is greater than the specific gravity of the first particle, when the reaction mixture is mixed with a liquid having a specific gravity greater than that of the first particle, less than that of the second particle and less than that of a particle complex composed of the first particle and the second particle, and allowed to stand undisturbed, a complex of the unreacted second probe, second probe and target substance and a complex of the first probe, second probe and target substance precipitate, while a complex of the unreacted first probe, first probe and target substance floats on top.

On the other hand, in the case the specific gravity of the second particle is less than the specific gravity of the first particle, when the reaction mixture is mixed with a liquid having a specific gravity less than that of the first particle, greater than that of the second particle, and greater than that of a particle complex composed of the first particle and the second particle, and allowed to stand undisturbed, a complex of the unreacted second probe, second probe and target substance and a complex of the first probe, second probe and target substance floats to the top, while a complex of the unreacted first probe, first probe and target substance precipitates.

At this time, since forces other than gravity and buoyancy do not act on the complex of the first probe, second probe and target substance, destruction and degradation do not occur in the complex of the first probe, second probe and target substance. Thus, according to the first invention, a complex of a first probe, a second probe and a target substance can be separated in the form of a precipitate or floating substance under mild conditions which do not cause destruction or degradation of said complex.

Although a complex of the first probe, second probe and target substance along with a complex of the unreacted second probe, second probe and target substance can be contained in the precipitate or floating substance, a complex of the unreacted first probe, first probe and target substance cannot be contained. This is important in the case the first probe is labeled (for example, when the first particle is a labeled particle), and the complex of the first probe, second probe and target substance are detected based on said label.

In the first invention, since the reaction among the first probe, second probe and target substance is carried out in a reaction solvent, the reaction solvent can be contained in the reaction mixture. In the case of mixing the reaction mixture containing a reaction solvent with the above-mentioned liquid, the specific gravity of the liquid varies as a result of mixing in the reaction solvent. Although this does not present a problem provided the above-mentioned conditions relating to specific gravity are still satisfied after the specific gravity has varied, if these conditions are not satisfied after specific gravity has varied, the complex of the first probe, second probe and target substance cannot be separated in the form of a precipitate or floating substance. Thus, the amount of reaction solvent contained in the reaction mixture is preferably reduced as much as possible to prevent variations in the specific gravity of the liquid as much as possible.

Therefore, in the first invention, the first particle and/or second particle is preferably a magnetic particle, and the reaction mixture is preferably obtained by reacting the first probe, the second probe and the target substance in a reaction solvent housed in a container, followed by removing the reaction solvent in a state in which a magnet is arranged in the vicinity of the outer wall of the container.

According to the present aspect, variations in specific gravity of the liquid can be prevented by reducing the amount of reaction solvent contained in the reaction mixture. In addition, according to the present aspect, since the complex of the first probe, second probe and target substance can be retained in the container by allowing the magnet to act on the magnetic particle when removing the reaction solvent, decreases in the amount of the complex contained in the reaction mixture can be prevented.

In the present aspect, the first particle is preferably a non-magnetic particle, and the second particle is preferably a magnetic particle. In this case, the complex of the unreacted second probe, second probe and target substance, and the complex of the first probe, second probe and target substance, can be retained in the container by the action of the magnet on the magnetic particle. On the other hand, since the complex of the unreacted first probe, first probe and target substance can be removed together with the reaction solvent, the amount of the complex of the unreacted first probe, first probe and target substance contained in the reaction mixture can be decreased. Thus, when forming a precipitate and floating substance by mixing the reaction mixture with the liquid, the possibility of the complex of the unreacted first probe, first probe and target substance contaminating the precipitate or floating substance containing the complex of the first probe, second probe and target substance can be reduced.

In order to solve the above-mentioned problems, a second invention provides a method for reacting a first probe having a first particle and a first binding substance immobilized on the first particle, a second probe having a second particle and a second binding substance immobilized on the second particle, and a target substance having a first portion able to be bound by the first binding substance and a second portion able to be bound by the second binding substance, wherein, the first particle and/or second particle is a magnetic particle, and the method comprises a first step in which the first probe, the second probe, the target substance and a reaction solvent are housed in a container, a second step in which a magnet is allowed to act on the magnetic particle within the container through a predetermined region of the outer wall of the container, a third step in which the action of the magnet is canceled, and a fourth step in which the second and third steps are repeated while changing the location of the predetermined region.

When the magnet acts on the magnetic particle in the container through a predetermined region of the outer wall of the container, molecules containing the magnetic particle (referring to a complex of the unreacted first probe and/or first probe and the target substance in the case the first particle is a magnetic particle, or referring to a complex of the unreacted second probe and/or second probe and the target substance in the case the second particle is a magnetic particle) move through the reaction solvent in the direction of the predetermined region. During this movement, a portion of the molecules containing the magnetic particle react by encountering other molecules (the unreacted first probe reacts with the target substance, the complex of the first probe and target substance reacts with the unreacted second probe, the unreacted second probe reacts with the target substance, and the complex of the second probe and target substance reacts with the unreacted first probe). The remainder of the molecules containing the magnetic particle ultimately move to the inner wall of the container without reacting with other molecules and adhere to the inner wall of the container.

If the action of the magnet is canceled before or after the molecules containing magnetic particle is adhered to the inner wall of the container, the molecules containing magnetic particle is again suspended in the reaction solvent and are subjected to the action of the magnet.

If the location of the predetermined region is changed and the magnet acts on the magnetic particle in the container through the predetermined location after this change, molecules containing magnetic particle move through the reaction solvent in the direction of the changed predetermined region. Thus, if the second and third steps are repeated while changing the location of the predetermined region, molecules containing magnetic particle move through the reaction solvent while changing the direction of movement thereof. As a result, the probability of molecules containing magnetic particle encountering other molecules increases, and reactions between molecules containing magnetic particle and other molecules can be accelerated. At this time, by regulating the amount of magnetism (magnetic charge) of the magnet to regulate the rate of movement of molecules containing magnetic particle, the first probe, second probe and,target substance can be reacted with a complex of the first probe, second probe and target substance under mild conditions which do not cause destruction or degradation of the complex.

In the second invention, the magnet is allowed to act by allowing the predetermined region and the magnet to approach each other in the second step, the action of the magnet is canceled by separating the predetermined region from the magnet in the third step, and the predetermined region and magnet are repeatedly allowed to approach and separate from each other while changing the location of the predetermined region in the fourth step.

According to the present aspect, the first probe, second probe and target substance can be reacted with a complex of the first probe, second probe and target substance under mild conditions which do not cause destruction or degradation of the complex by regulating the amount of magnetism (magnetic charge) of the magnet, the distance between the container and the magnet, and the rates of approach and separation.

In the second invention, the location of the predetermined region is preferably changed in the circumferential or lengthwise direction of the container.

According to the present aspect, since molecules containing magnetic particle move through the reaction solvent in various directions, the probability of molecules containing magnetic particle encountering other molecules can be further increased, and the reaction between molecules containing magnetic particle and other molecules can be further accelerated.

In order to solve the above-mentioned problems, a third invention provides a method for detecting a target substance having a first portion able to be bound by a first binding substance and a second portion able to be bound by a second binding substance, using a first probe having a first particle and the first binding substance immobilized on the first particle, and a second probe having a second particle and the second binding substance immobilized on the second particle, the method comprising a first step in which a labeled particle is selected for the first particle and a particle having a specific gravity greater than that of the first particle is selected for the second particle, a second step in which the first probe, the second probe and the target substance are reacted to obtain a reaction mixture, a third step in which the reaction mixture is mixed with a liquid having a specific gravity greater than that of the first particle, less than that of the second particle, and less than that of a particle complex composed of the first particle and the second particle, followed by allowing to stand undisturbed until a precipitate and a floating substance are formed, and a fourth step in which the complex of the first probe, second probe and target substance contained in the precipitate is detected based on the label of the first particle.

In order to solve the above-mentioned problems, a fourth invention provides a method for detecting a target substance having a first portion able to be bound by a first binding substance and a second portion able to be bound by a second binding substance, using a first probe having a first particle and the first binding substance immobilized on the first particle, and a second probe having second particle and the second binding substance immobilized on the second particle, the method comprising a first step in which a labeled particle is selected for the first particle and a particle having a specific gravity less than that of the first particle is selected for the second particle, a second step in which the first probe, the second probe and the target substance are reacted to obtain a reaction mixture, a third step in which the reaction mixture is mixed with a liquid having a specific gravity less than that of the first particle, greater than that of the second particle, and greater than that of a particle complex composed of the first particle and the second particle, followed by allowing to stand undisturbed until a precipitate and a floating substance are formed, and a fourth step in which the complex of the first probe, the second probe and the target substance contained in the floating substance is detected based on the label of the first particle.

According to the third and fourth inventions, a complex of a first probe, a second probe and a target substance can be separated in the form of a precipitate or floating substance under mild conditions which do not cause destruction of degradation of said complex in the same manner as the first invention. Although a complex of the unreacted second probe, second probe and target substance can be contained in the precipitate or floating substance together with a complex of the first probe, second probe and target substance, a complex of the unreacted first probe, first probe and target substance cannot be contained. Thus, a complex of the first probe, second probe and target substance contained in the precipitate or floating substance can be detected based on the label of the first particle.

In the third and fourth inventions, a magnetic particles is preferably selected for the first particle and/or second particle in the first step, and the reaction mixture is preferably obtained by reacting the first probe, the second probe and the target substance in a reaction solvent housed in a container, followed by removing the reaction solvent in a state in which a magnet is arranged in the vicinity of the outer wall of the container in the second step.

According to the present aspect, variations in specific gravity of the liquid can be prevented by reducing the amount of reaction solvent contained in the reaction mixture. In addition, according to the present aspect, since the complex of the first probe, second probe and target substance can be retained in the container by allowing the magnet to act on the magnetic particle when removing the reaction solvent, decreases in the amount of the complex contained in the reaction mixture can be prevented.

In the present aspect, a non-magnetic particle is preferably selected for the first particle, and a magnetic particle is preferably selected for the second particle in the first step. As a result, the complex of the unreacted second probe, second probe and target substance, and the complex of the first probe, second probe and target substance, can be retained in the container. On the other hand, since the complex of the unreacted first probe, first probe and target substance can be removed together with the reaction solvent, the amount of the complex of the unreacted first probe, first probe and target substance contained in the reaction mixture can be decreased. Thus, when forming a precipitate and floating substance by mixing the reaction mixture with the liquid, the possibility of the complex of the unreacted first probe, first probe and target substance contaminating the precipitate or floating substance containing the complex of the first probe, second probe and target substance can be reduced, and the detection accuracy of the complex of the first probe, second probe and target substance contained in the precipitate or floating substance can be improved.

In the third and fourth inventions, a magnetic particle is preferably selected for the first particle and/or second particle in the first step, and the first probe, second probe and target substance are preferably reacted according to the method of the second invention in the second step.

According to the present aspect, a first probe, a second probe and a target substance can be reacted under mild conditions which do not cause destruction or degradation of a complex of the first probe, second probe and target substance in the same manner as the second invention.

In the present aspect, the reaction mixture is preferably obtained by reacting the first probe, the second probe and the target substance followed by removing the reaction solvent in the state in which a magnet is arranged in the vicinity of the outer wall of the container in the second step. As a result, the amount of reaction solvent contained in the reaction mixture can be reduced, and variations in the specific gravity of the liquid can be prevented. In addition, since a complex of a first probe, second probe and target substance can be retained in the container by allowing a magnet to act onmagnetic particle when removing the reaction solvent, decreases in the amount of said complex contained in the reaction mixture can be prevented.

In the present aspect, a non-magnetic particle is preferably selected for the first particle and a magnetic particle is preferably selected for the second particle in the first step. As a result, a complex of the unreacted second probe, second probe and target substance and a complex of the first probe, second probe and target substance can be retained in the container. On the other hand, since a complex of the unreacted first probe, first probe and target substance can be removed together with the reaction solvent, the amount of the complex of the unreacted first probe, first probe and target substance contained in the reaction mixture can be decreased. Thus, when forming a precipitate and floating substance by mixing the reaction mixture with the liquid, the possibility of the complex of the unreacted first probe, first probe and target substance contaminating the precipitate or floating substance containing the complex of the first probe, second probe and target substance can be reduced, and the detection accuracy of the complex of the first probe, second probe and target substance contained in the precipitate or floating substance can be improved. In the third and fourth inventions, a magnetic particle is preferably selected for the second particle in the first step, and the first probe is reacted with a complex of the second probe and the target substance obtained by mixing a sample containing the target substance and the second probe, and recovering the complex of the second probe and the target substance by controlling magnetic force in the second step.

According to the present aspect, since impurities contained in a sample can be prevented from contaminating a reaction mixture, the detection accuracy of a target substance can be improved.

According to the present invention, a method is provided for separating a complex of a first probe, a second probe and a target substance from a reaction mixture obtained by reacting a first probe having a first particle (for example, labeled particle) and a first binding substance (for example, a first oligonucleotide) immobilized on the first particle, a second probe having a second particle (for example, magnetic particle) and a second binding substance (for example, a second oligonucleotide) immobilized on the second particle, and a target substance (for example, a target nucleic acid) , under mild conditions which do not cause destruction or degradation of said complex.

In addition, according to the present invention, a method is provided for reacting a first probe having a first particle (for example, labeled particle) and a first binding substance (for example, a first oligonucleotide) immobilized on the first particle, a second probe having a second particle (for example, magnetic particle) and a second binding substance (for example, a second oligonucleotide) immobilized on the second particle, and a target substance (for example, a target nucleic acid), under mild conditions which do not cause destruction or degradation of a complex of the first probe, the second probe and the target substance.

Moreover, according to the present invention, a method is provided for detecting a target substance (for example, a target nucleic acid) using a first probe having a first particle (for example, labeled particle) and a first binding substance (for example, a first oligonucleotide) immobilized on the first particle, and a second probe having a second particle (for example, magnetic particle) and a second binding substance (for example, a second oligonucleotide) immobilized on the second particle, under mild conditions which do not cause destruction or degradation of a complex of the first probe, the second probe and the target substance.

### BRIEF DESCRIPTION OF THE DRAWINGS

FIG. 1 is a schematic cross-sectional view of a dispenser used in an embodiment of the method of the present invention;
FIG. 2 is a schematic cross-sectional view showing each step (steps a to d) of the same embodiment;
FIG. 3 is a schematic cross-sectional view representing each step (steps e to h) of the same embodiment;
FIG. 4 is a schematic cross-sectional view representing each step (steps i to k) of the same embodiment;
FIG. 5 is a schematic cross-sectional view representing each step (steps 1 to n) of the same embodiment;
FIG. 6 is a schematic cross-sectional view representing each step (steps o to q) of the same embodiment;
FIG. 7 is a schematic cross-sectional view representing each step (steps r to u) of the same embodiment;
FIG. 8A is a schematic overhead view showing an embodiment of a method for allowing a magnet to act on magnetic particles in a container through a predetermined region while changing the location of the predetermined region of the outer wall of the container in the circumferential direction of the container, while FIG. 8B is a schematic overhead view showing another embodiment of the same method;
FIG. 9 is a schematic overhead view showing another embodiment of a method for allowing a magnet to act on magnetic particles in a container through a predetermined region while changing the location of the predetermined region of the outer wall of the container in the circumferential direction of the container;
FIG. 10A is a schematic overhead view showing an embodiment of a method for allowing a magnet to act on magnetic particles in a container through a predetermined region while changing the location of the predetermined region of the outer wall of the container in the lengthwise direction of the container, while FIG. 10B is a perspective view showing another embodiment of the same method; and
FIG. 11 is a schematic drawing showing the bound state of a probe and a target nucleic acid.

### BEST MODE FOR CARRYING OUT THE INVENTION

There are no particular limitations on the various liquids (such as the reaction solvent and washing liquid) used in the method of the present invention provided they are insoluble, specific examples of which include polymers obtained by polymerizing one or more types of aromatic vinyl compounds such as styrene, chlorostyrene, chloromethylstyrene, α-methylstyrene, divinylbenzene, sodium styrene sulfonate, (meth) acrylic acid, methyl (meth) acrylate, ethyl (meth)acrylate, n-butyl (meth)acrylate, 2-hydroxyethyl (meth)acrylate, polyoxyethylene (meth)acrylate, glycidyl (meth)acrylate, ethylene glycol di(meth)acrylic acid ester, tribromophenyl (meth)acrylate, tribromopropyl acrylate, (meth)acrylonitrile, (meth)acrolein, (meth)acrylamide, methylene-bis(meth)acrylamide, butadiene, isoprene, vinyl acetate, vinyl pyridine, N-vinylpyrrolidone, vinyl chloride and vinyl bromide, esters or amides of α,β-unsaturated carboxylic acids, α,β-unsaturated nitrile compounds, halogenated vinyl compounds, conjugated diene compounds or lower fatty acid vinyl esters; crosslinked polysaccharides such as agarose, dextran, cellulose and carboxymethyl cellulose; crosslinked proteins such as methylated albumin, gelatin, collagen and casein; inorganic materials such as glass and ceramics; metals such as iron and silicon; and, compound materials thereof.

There are no particular limitations on the substance (magnetic substance) which demonstrates magnetism contained in the magnetic particles, specific examples of which include metals such as Fe, Ni, Co, Gd, Tb and Dy; alloys and intermetallic compounds such as Fe-Ni, Fe-Co, Fe-Al, Fe-Si-Al, Fe-Ni-Mo, Al-Ni-Co, Sm-Co and Nd-Fe-B; and, ferrite compounds such as Fe₂O₃, Fe₂O₃-NiO-ZnO, Fe₂O₃-CuO-ZnO and Fe₂O₃-MnO-ZnO.

Ferromagnetic particles or paramagnetic particles having a large magnetic susceptibility, for example, can be used for the magnetic particles. These magnetic particles preferably have low residual magnetization to improve dispersibility in liquid after being acted on by a magnet, and preferably have large saturation magnetization to improve reactivity with the magnet. In addition, although the magnetic particles may be anisotropic, they are preferably isotropic.

There are no particular limitations on the shape of the particles, and as a specific example, the particles may be spherical. In addition, there are no particular limitations on the particle diameter of the particles provided that the particles are able to precipitate or float due to the effects of gravity or buoyancy in a liquid allowed to stand undisturbed, a specific example of the range of which is a diameter of about 0.1 to 100 µm.

There are no particular limitations on the label of the labeled particles, specific examples of which include fluorescent labels obtained from fluorescent compounds such as Marine Blue, Cascade Blue, Cascade Yellow, Fluorescein, Rhodamine, Phycoerythrin, CyChrome, PerCP, Texas Red, Allophycocyanin and PharRed, Cy-based dyes such as Cy2, Cy3, Cy3.5, Cy5 and Cy7, Alexa-based dyes such as Alexa-488, Alexa-532, Alexa-546, Alexa-633 and Alexa-680, and BODIPY-based dyes such as BODIPY FL and BODIPY TR; chemifluorescent labels obtained from chemifluorescent compounds such as luminol, lucigenin and acridium ester; enzyme labels obtained from enzymes such as alkaline phosphatase and horseradish peroxidase; and, bioluminescent labels obtained from bioluminescent compounds such as luciferase and luciferin. These labels can be detected in accordance with ordinary methods.

There are no particular limitations on the target substance, specific examples of which include biological substances such as nucleic acids, proteins, antigens, antibodies, enzymes and sugars. Furthermore, DNA, RNA and analogs and derivatives thereof (such as peptide nucleic acids (PNA) and phosphorothioate DNA) are included in nucleic acids.

There are no particular limitations on the binding substances provided they is able to bind with the target substance, specific examples of which include biological substances such as nucleic acids, proteins, antigens, antibodies, enzymes and sugars. Specific examples of combinations of target substances and binding substances include nucleic acids and complementary nucleic acids, receptor proteins and ligands, enzymes and substrates, and antibodies and antigens.

Since the first binding substance and the second binding substance bind to different portions of the target substance (first and second portions), the first probe and the second probe are able to bind simultaneously to the target substance.

The binding substances preferably bind specifically to the target substance. "Bind specifically" refers to not binding to a portion other than the predetermined portion of the target substance. In the case the target substance and binding substances are nucleic acids, "bind specifically" refers to hybridizing under stringent conditions, and examples of stringent conditions include conditions of 2°C, 2xSSC and 0.1% SDS, and preferably 65°C, 0.1xSSC and 0.1% SDS.

There are no particular limitations on the number of binding substances immobilized on a single particle, and although one or a plurality of binding substances may be immobilized, normally a plurality are immobilized.

Immobilization of a binding substance on the particles can be carried out by various binding modes. Specific examples of binding modes include specific interaction between streptoavidin or avidin and biotin, hydrophobic interaction, magnetic interaction, polar interaction, covalent bond formation (such as amide bonds, disulfide bonds or thioether bonds), and crosslinking using a crosslinking agent. The particle surface or binding substance can be subjected to suitable chemical modification using a known technology so as to enable immobilization by these binding modes.

In addition to specific interaction of streptoavidin or avidin and biotin, the binding substance can also be immobilized on the particles by using a specific interaction such as that between maltose-binding protein and maltose, polyhistidine peptide and nickel, cobalt or other metal ions, glutathione-S-transferase and glutathione, calmodulin and calmodulin-binding peptide, ATP-binding protein and ATP, nucleic acid and complementary nucleic acid, receptor protein and ligand, enzyme and substrate, antibody and antigen or IgG and protein A.

In the case of using the specific interaction between avidin or streptoavidin and biotin, a binding substance into which biotin has been introduced (such as a biotinated nucleic acid obtained by carrying out PCR using a primer having a biotinated 5'-end) can be bound to particles coated with avidin or streptoavidin.

In the case of using the formation of covalent bonds, covalent bonds can be formed by using functional groups present on the particle surface or binding substance. Specific examples of functional groups capable of being covalently bonded include carboxyl groups, amino groups and hydroxyl groups. For example, in the case of carboxyl groups being present on the surface of the particles, after activating the carboxyl groups with a carbodiimide such as 1-ethyl-3-(3-dimethylaminopropyl)-3-ethyl carbodiimide hydrochloride (EDC), the particles and binding substance can be bound by amide bonds by reacting with the amino groups present in the binding substance. In addition, in the case amino groups are present on the surface of the particles, after converting the amino groups to carboxyl groups using a cyclic acid anhydride such as succinic anhydride, the particles and binding substance can be bound by amide bonds by reacting with amino groups present in the binding substance.

In the case of using crosslinking with a crosslinking agent, various crosslinking agents able to react with a functional group of the substance to be crosslinked can be used. Specific examples of the crosslinking agents include multifunctional reagents such as a bifunctional reagent and trifunctional reagent. Specific examples of these multifunctional reagents include N-succinimidyl(4- iodoacetyl)aminobenzoate (SIAB), dimaleimide, dithio-bis- nitrobenzoic acid (DTNB), N-succinimidyl-S-acetyl-thioacetate (SATA), N-succinimidyl-3-(2-pyridyldithiio)propionate (SPDP), succinimidyl-4-(N-maleimidomethyl)cyclohexane-1-carboxylate (SMCC) and 6-hydrazinonicotimide (HYNIC).

There are no particular limitations on the reaction solvent used when reacting the first probe, second probe and target substance provided they do not inhibit the reaction, and an example of such a reaction solvent is a buffer. The composition of the reaction solvent can be suitably adjusted corresponding to the types of target substance, binding substances and so on.

When reacting the first probe, second probe and target substance, there are no particular limitations on the order in which the first probe, second probe, target substance and reaction solvent are placed in the container, and they may be added separately in any arbitrary order, or two or more types may be arbitrarily combined and added simultaneously. When simultaneously adding the first probe and target substance, the first probe and target substance may be in a bound state or unbound state. This applies similarly to the case of adding the second probe and target substance simultaneously.

The reaction conditions when reacting the first probe, second probe and target substance (for example, reaction temperature and reaction time) can be suitably adjusted corresponding to the types of target substance, binding substances and so on.

There are no particular limitations on the liquid having a specific gravity greater than that of the first particles, less than that of the second particles and less than that of a complex composed of the first particles and second particles, or on a liquid having a specific gravity less than that of the first particles, greater than that of the second particles, and greater than that of a complex composed of the first particles and second particles, provided the complex of the first probe, second probe and target substance is able to exist in a stable state, specific examples of which include a water-soluble nonionic polymer solution (e.g., Ficoll solution (Amersham Pharmacia Biotech)), an aqueous sucrose solution, an aqueous polyethylene glycol solution and an aqueous glycerol solution. Ficoll solution is particularly preferable among these solutions. Ficoll is a copolymer of sucrose and epichlorhydrin (molecular weight: approx. 400,000), and aqueous solutions of various specific gravities can be prepared by changing the concentration of the Ficoll. The liquid may be composed of one type of liquid, or may be a mixture of two or more types of liquids. The specific gravity of the liquid can be suitably adjusted according to the type of liquid, concentration and so on.

The specific gravity of the particles can be suitably adjusted according to the type of composite materials, contents thereof and so on. In the case of selecting labeled non-magnetic particles for the first particles and selecting magnetic particles having a specific gravity greater than that of the first particles for the second particles, the specific gravity of the first particles can be made to be less than 3 (specific range of, for example, 1.03 to 3), and the specific gravity of the second particles can be made to be, for example, 3 or more.

The specific gravity of the particles is calculated based on mass and volume. In addition, the specific gravity of a complex composed of the first particles and the second particles can be calculated based on the total mass and total volume of the first particles and second particles. Namely, the specific gravity of a complex composed of the first particles and second particles can be calculated as the ratio between the total mass of the first particles and second particles and a standard substance of the same volume as the total volume of the first particles and second particles (normally water at 4°C).

When mixing the reaction mixture with a liquid having a specific gravity greater than that of the first particles, less than that of the second particles and less than that of a complex composed of the first particles and second particles, or a liquid having a specific gravity less than that of the first particles, greater than that of the second particles, and greater than that of a complex composed of the first particles and second particles, the reaction mixture may be added to said liquid, or said liquid may be added to the reaction mixture.

There are no particular limitations on the type of magnet, and a permanent magnet or temporary magnet may be used. Examples of permanent magnets include aluminum-nickel-cobalt magnets, ferrite magnets and rare earth metal magnets, while an example of a temporary magnet is an electromagnet.

The phrase "predetermined region of the outer wall of the container" refers to a single predetermined region or a plurality of different predetermined regions. Thus, "the magnet is allowed to act on the magnetic particles within the container through a predetermined region of the outer wall of the container" includes the case of allowing the magnet to act through a single predetermined region and the case of allowing the magnet to act through a plurality of different predetermined regions. In the case of allowing the magnet to act through a plurality of different predetermined regions, different magnets may be used for each region or the same magnet may be used for each region. There are no particular limitations on the location, surface area or shape and so on of the predetermined region(s). The predetermined region before changing and the predetermined region after changing may be continuous or discontinuous. The predetermined region before changing and the predetermined region after changing may or may not share a common portion. There are no particular limitations on the mode of change in the location of the predetermined region, and examples include a change in the circumferential direction of the container, in the lengthwise direction of the container, and a combined direction thereof.

There are no particular limitations on the method for allowing the magnet to act on the magnetic particles in the container through a predetermined region of the outer wall of the container, and for example, the magnet can be allowed to act by allowing the magnet and the predetermined region to approach each other. At this time, the magnet used may be a permanent magnet or a temporary magnet. In the case of using a temporary magnet, the magnet may be in magnetized state prior to allowing it to approach the predetermined region, or it may be magnetized after approaching the predetermined region. The distance between the predetermined region and magnet when in close proximity to each other can be suitably adjusted corresponding to the amount of magnetism (magnetic charge) of the magnet. When the predetermined reaction and magnet are allowed to approach each other, the magnet may be moved with the container is in a stationary state, the container may be moved with the magnet in a stationary state, or both the magnet and container may be moved together (for example, in the same direction or opposite directions).

There are no particular limitations on the method for canceling the action of the magnet, and for example, the action of the magnet can be canceled by separating the predetermined region and the magnet. In the case of using a temporary magnet, the action of the magnet can be canceled by demagnetizing the magnet while in close proximity with the predetermined region. The distance between the predetermined region and magnet when separated can be suitably adjusted corresponding to the amount of magnetism (magnetic charge) of the magnet. When separating the predetermined region and magnet, the magnet may be moved with the container in a stationary state, the container may be moved with the magnet in a stationary state, or both the magnet and the container may be moved together (for example, in the same direction or opposite directions).

The same magnet may be used before and after the change in the location of the predetermined region, different magnets may be used, or different portions of the same magnet may be used.

Although there are no particular limitations on the number of times the action of the magnet through the predetermined region of the outer wall of the container is activated and canceled, it is preferably a number of times which allows a complex of the first probe, second probe and target substance to be adequately formed.

Although there are no particular limitations on the location of the magnet when removing the reaction solvent with the magnet arranged in close proximity to the outer wall of the container, it is preferably near the outer wall at the lower portion or bottom of the container. There are no particular limitations on the distance between the magnet and the outer wall of the container provided the magnet is able to act on the magnetic particles in the container, and this distance can be suitably adjusted corresponding to the amount of magnetism (magnetic charge) of the magnet.

In addition to ordinarily used containers such as bottomed cylindrical containers, bottomless cylindrical containers (such as a pipette tip) can also be used for the container. A liquid can be retained in an aspirated state in a bottomless cylindrical container.

The container is preferably transparent or translucent. This enables light emitted from inside the container (such as fluorescence or chemiluminescence) to be detected outside the container. Examples of transparent or translucent materials include plastic and glass.

When detecting a complex of the first probe, second probe and target substance contained in a precipitate or floating substance, although the complex may be detected without recovering the precipitate or floating substance, the complex is preferably detected after recovering the precipitate or floating substance. The precipitate or floating substance may be recovered together with the liquid. A precipitate can be recovered by, for example, removing the liquid supernatant followed by recovering the residual liquid. A floating substance can be recovered, for example, in the form of the liquid supernatant.

There are no particular limitations on the sample containing the target substance, specific examples of which include biological materials such as blood, serum, plasma, excrement, cerebrospinal fluid, seminal fluid, saliva, cell lysate, tissue lysate, cell culture or tissue culture.

There are no particular limitations on the method for recovering a complex of the second probe (in which the second particles of the second probe are magnetic particles) and the target substance by controlling magnetic force provided it is able to control the behavior of the magnetic particles, and the complex can be recovered by, for example, arranging a magnet in the pipette tip of a dispenser which aspirates and discharges liquid from the container to allow the magnet to act and retain the complex in the liquid aspirated into the pipette tip on the inner walls of the pipette tip, while on the other hand, causing the complex to be released from the inner walls of the pipette tip by making it no longer susceptible to the action of the magnet, and then discharging the complex outside the pipette tip along with the liquid.

The following provides an explanation of an embodiment of a method for detecting a target substance as claimed in the present invention based on the drawings.

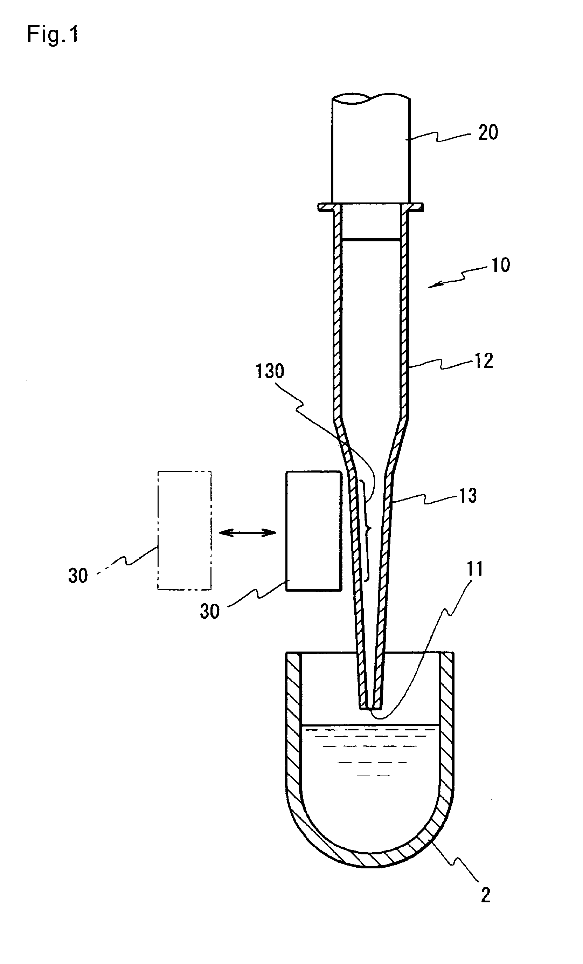

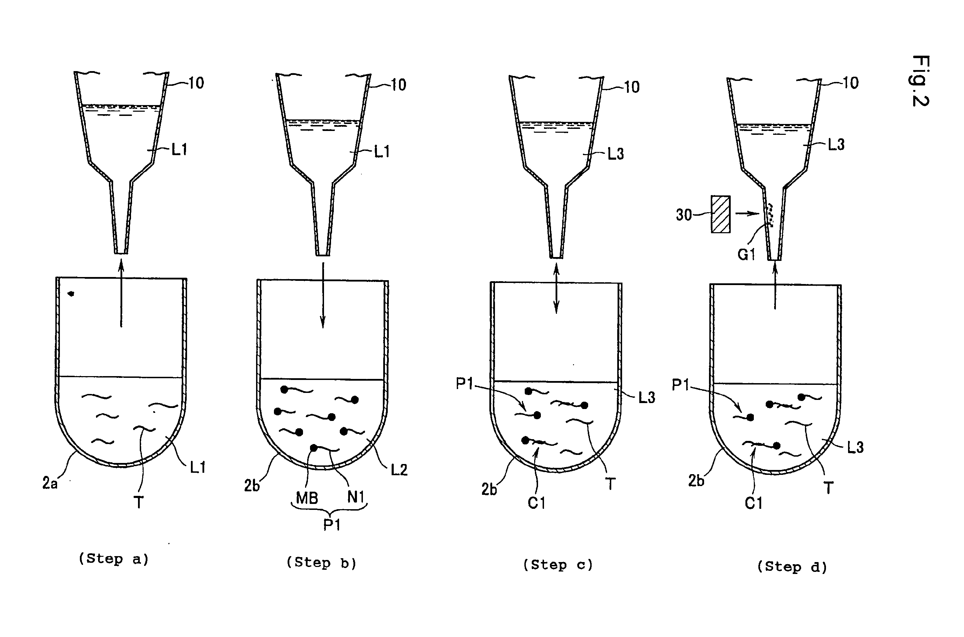

Fig. 1 is a schematic drawing of a dispenser used in the present embodiment, while Figs. 2 to 7 are schematic cross-sectional views representing each step of the present embodiment.

As shown in Fig. 1, the dispenser used in the present embodiment is provided with a pipette tip 10, a nozzle 20 removably attached to the opening to the opening in the upper end of pipette tip 10, a liquid aspiration and discharge device (not shown), which aspirates liquid into pipette tip 10 or discharges liquid from pipette tip 10 by increasing or decreasing pressure within pipette tip 10 through nozzle 20, a pipette tip movement device (not shown), which moves pipette tip 10 in a desired direction (such as vertically or horizontally), a magnet 30, a magnet movement device (not shown), which moves magnet 30 either towards or away from pipette tip 10, and a control device (not shown), which controls the operation of each device.

Pipette tip 10 has a tip 11, which is inserted into a container 2, a large-diameter reservoir 12 for retaining a liquid, and a narrow-diameter liquid pathway 13 continuous with tip 11 and reservoir 12. Liquid pathway 13 has a separation region 130 which is affected by the action of magnet 30 when in close proximity to pipette tip 10. The liquid aspiration and discharge device may be, for example a syringe, while magnet 30 may be, for example, a permanent magnet.

In the present embodiment, as shown in Fig. 2 (Step a), liquid sample L1 in container 2a is first aspirated into pipette tip 10. Liquid sample L1 is, for example, a biological material such as blood, serum, plasma, excrement, cerebrospinal fluid, seminal fluid, saliva, cell lysate, tissue lysate, cell culture or tissue culture. Various impurities (such as nucleic acids other than a target nucleic acid, proteins and lipids) (not shown) are contained in liquid sample L1 in addition to target nucleic acid T.

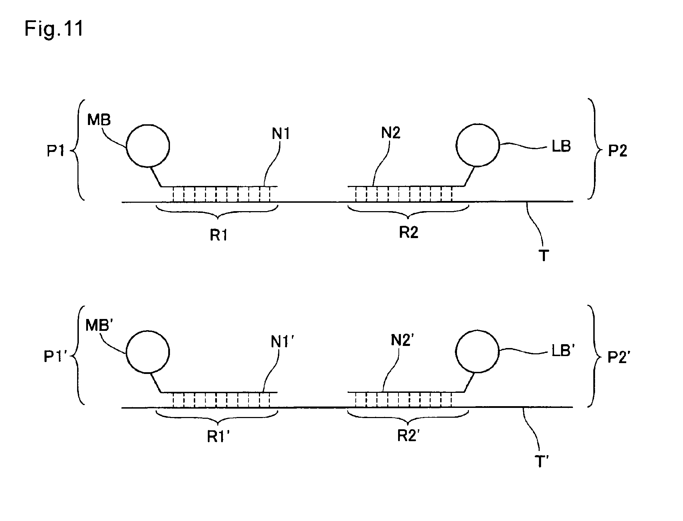

Next, as shown in Fig. 2 (Step b), pipette tip 10 is moved, and liquid sample L1 in pipette tip 10 is discharged into container 2b. A suspension L2 of a probe P1 is housed in container 2b, and as a result of liquid sample L1 in pipette tip 10 being discharged into container 2b, a mixture L3 of sample liquid L1 and suspension L2 is prepared. As shown in Fig. 2 and Fig. 11, probe P1 is composed of magnetic particles MB and an oligonucleotide N1 immobilized on magnetic particles MB. As shown in Fig. 11, oligonucleotide N1 is composed of a nucleotide sequence complementary to region R1 of target nucleic acid T, and is able to specifically hybridize with region R1 of target nucleic acid T.

Next, as shown in Fig. 2 (Step c), mixture L3 is repeatedly aspirated into and discharged from pipette tip 10. As a result, probe P1 and target nucleic acid T react, and a hybrid C1 of probe P1 and target nucleic acid T is formed.

Next, as shown in Fig. 2 (Step d), mixture L3 in container 2b is aspirated into pipette tip 10. At this time, as shown in Fig. 2 (Step d), magnet 30 moves in the direction which causes it to approach pipette tip 10. Thus, when mixture L3 passes through liquid pathway 13 of pipette tip 10, the molecules containing magnetic particles MB (unreacted probe P1 and hybrid C1) are captured on the inner walls of liquid pathway 13 by the action of magnet 30, and an aggregate G1, containing unreacted probe P1 and hybrid C1, is formed on the inner walls of liquid pathway 13 affected by the action of magnet 30.

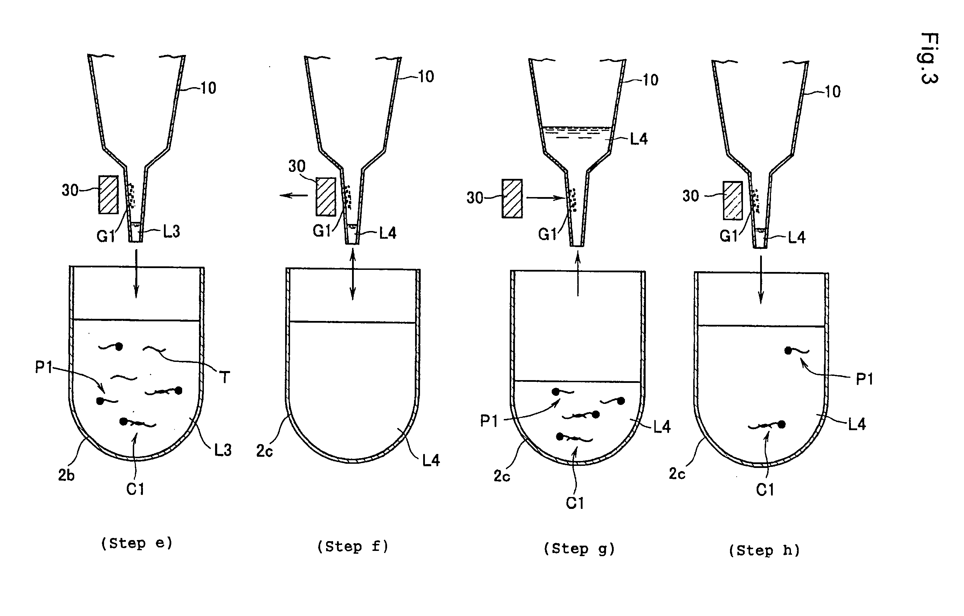

Next, as shown in Fig. 3 (Step e), mixture L3, excluding aggregate G1, is discharged from pipette tip 10 into container 2b. At this time, aggregate G1 is retained in a state of being adhered to the inner walls of liquid pathway 13 of pipette tip 10 even if mixture L3 is discharged.

Next, as shown in Fig. 3 (Step f), pipette tip 10 moves, and washing liquid L4 in container 2c is repeatedly aspirated into and discharged from pipette tip 10. At this time, as shown in Fig. 3 (Step f), magnet 30 moves in the direction in which it moves away from pipette tip 10 to cancel the retained state of aggregate G1. Thus, unreacted probe P1 and hybrid C1 contained in aggregate G1 are dispersed and washed by washing liquid L4.

Next, as shown in Fig. 3 (Step g), washing liquid L4 in container 2c is aspirated into pipette tip 10. As shown in Fig. 3 (Step g), magnet 30 again moves in the direction in which approaches pipette tip 10. Thus, when washing liquid L4 passes through liquid pathway 13 of pipette tip 10, molecules containing magnetic particles MB (unreacted probe P1 and hybrid C1) are captured on the inner walls of liquid pathway 13 due to the action of magnet 30, and aggregate G1, which contains unreacted probe P1 and hybrid C1, is formed on the inner walls of liquid pathway 13 affected by the action of magnet 30.

Next, as shown in Fig. 3 (Step h), washing liquid L4, excluding aggregate G1, is discharged from pipette tip 10 into container 2c. At this time, aggregate G1 is retained in a state of being adhered to the inner walls of liquid pathway 13 of pipette tip 10 even if washing liquid L4 is discharged.

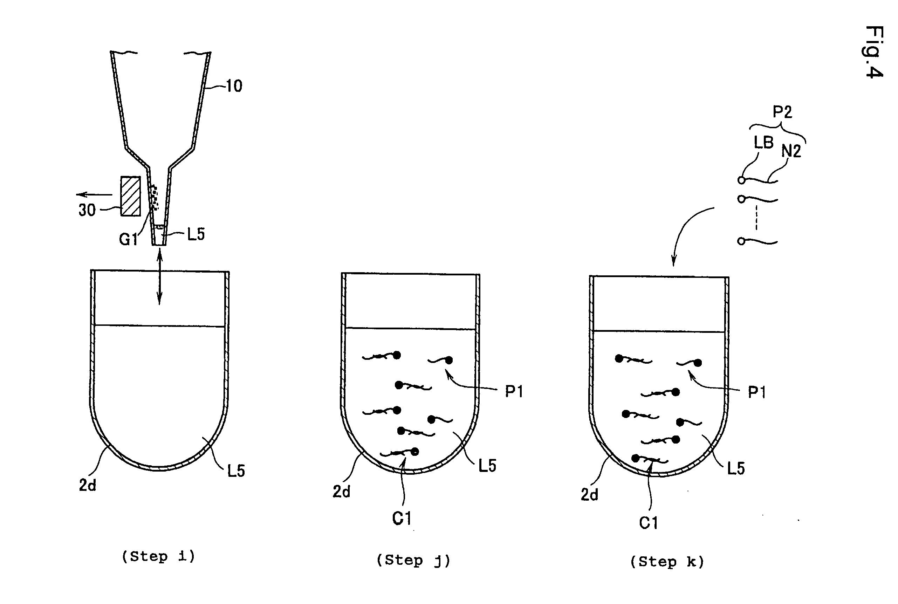

Next, as shown in Fig. 4 (Step i), pipette tip 10 is moved, and liquid L5 in container 2d is repeatedly aspirated into and discharged from pipette tip 10. At this time, as shown in Fig. 4 (Step i), magnet 30 moves in the direction in which it moves away from pipette tip 10, and the retained state of aggregate G1 is canceled. Thus, unreacted probe P1 and hybrid C1 contained in aggregate G1 are dispersed in liquid L5. Liquid L5 is, for example, a buffer.

Thus, as shown in Fig. 4 (Step j), liquid L5, which contains unreacted probe P1 and hybrid C1, but contains hardly any other impurities, is prepared in container 2d. As a result of liquid L5 containing hardly any impurities, the detection accuracy of target nucleic acid T can be improved. Furthermore, although not shown in the drawings, unreacted target nucleic acid T can also be contained in liquid 5.

Next, as shown in Fig. 4 (Step k), probe P2 is mixed into liquid L5 in container 2d. As shown in Figs. 4 and 11, probe P2 is composed of labeled non-magnetic particles LB and oligonucleotide N2 immobilized on labeled non-magnetic particles LB. As shown in Fig. 11, oligonucleotide N2 is composed of a nucleotide sequence complementary to region R2 of a target nucleic acid, and is able to specifically hybridized with region R2. Particles having a specific gravity less than that of magnetic particles MB are selected for labeled non-magnetic particles LB.

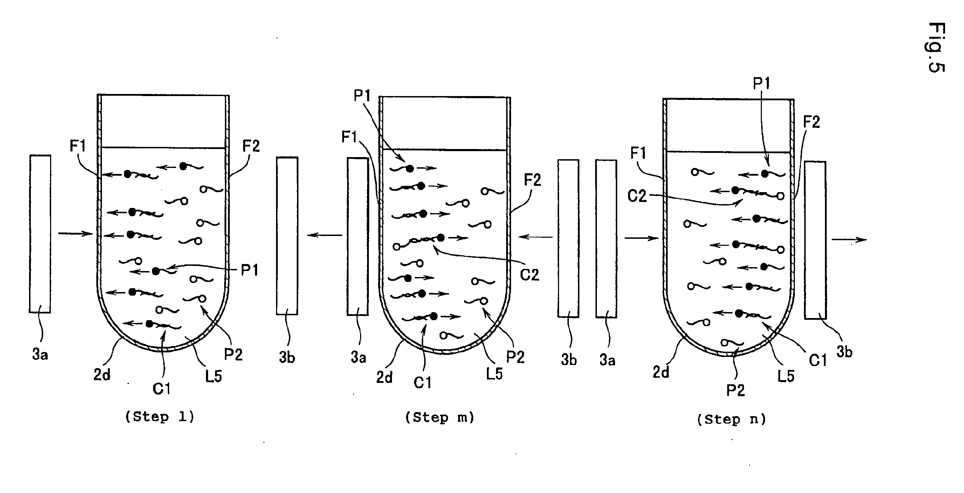

Furthermore, two magnets 3a and 3b are arranged in a state separated from the outer wall of container 2d around container 2d (see Fig. 5 (Step 1)). Since magnets 3a and 3b are arranged in a state separated from the outer wall of container 2d, the action of magnets 3a and 3b does not affect magnetic particles MB in container 2d.

Next, a shown in Fig. 5 (Step 1), magnet 3a, which is separated from region F1 of the outer wall of container 2d, is made to approach region F1. At this time, as shown in Fig. 5 (Step 1), magnet 3b is still separated from region F2 of the outer wall of container 2d. When magnet 3a approaches region F1, magnet 3a acts on magnetic particles MB, and hybrid C1 moves through liquid L5 in the direction of region F1. A portion of hybrid C1 encounters and reacts with probe 2 during this movement, and hybrid C2 consisting of probe P1, probe 2 and target nucleic acid T is formed. The remainder of hybrid C1 ultimately moves to the inner walls of container 2d without encountering probe 2, and is adhered to the inner walls of container 2d.

Next, as shown in Fig. 5 (Step m), magnet 3b separated from region F2 is made to approach region F2 while after moving away or while moving away magnet 3a from region F1. When magnet 3a is moved away from region F1, hybrid C1 again is suspended in liquid L5. When magnet 3b is made to approach region F2, magnet 3b acts on magnetic particles MB, and hybrid C1 suspended in liquid L5 moves through liquid L5 in the direction of region F2. A portion of hybrid C1 encounters and reacts with probe P2 during that movement, resulting in the formation of hybrid C2. The remainder of hybrid C1 ultimately moves to the inner walls of container 2d without encountering probe P2, and is adhered to the inner walls of container 2d.

Next, as shown in Fig. 5 (Step n), magnet 3a, which has been separated from region F1, is made to approach region F1 after moving away or while moving away magnet 3b from region F2. In the same manner as Step m, a portion of hybrid C1 encounters and reacts with probe P2 during movement through liquid L5, resulting in the formation of hybrid C2. The remainder of hybrid C1 ultimately moves to the inner walls of container 2d without encountering probe P2, and is adhered to the inner walls of container 2d.

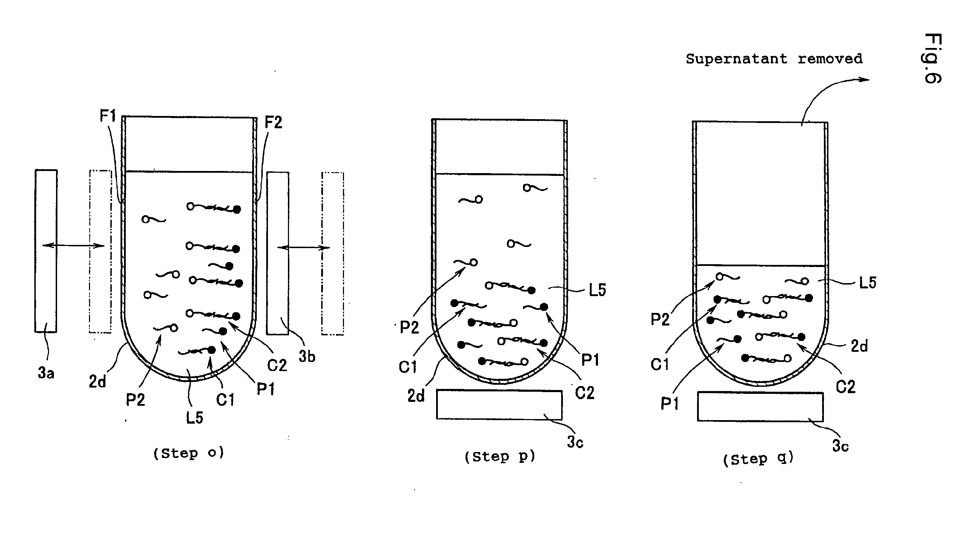

Next, as shown in Fig. 6 (Step o), steps m and n are repeated a predetermined number of times. Although there are no particular limitations on the number of times steps m and n are repeated, they are preferably repeated a number of times which is sufficient for formation of hybrid C2. The detection accuracy of target nucleic acid T can be improved by adequately forming hybrid C2.

In Steps 1 to o, hybrid C2 is formed under mild conditions which do not cause destruction or degradation of hybrid C2 by adjusting the amounts of magnetism (magnetic charge) of magnets 3a and 3b, the distance between container 2d and magnet 3a or 3b, and the rates at which magnets 3a and 3b are made to approach and move away from container 2d.

Next, as shown in Fig. 6 (Step p), a magnet 3c is arranged in close proximity to the outer wall at the bottom of container 2d. Due to the action of magnet 3c on magnetic particles MB, hybrid C2 moves toward the bottom of container 2d together with unreacted probe P1 and hybrid C1, and is ultimately maintained in an adhered state on the inner wall at the bottom of container 2d. On the other hand, since unreacted probe P2 does not contain magnetic particles MB in molecules thereof, unreacted probe P2 is not affected by the action of magnet 3c, and remains suspended in liquid L5.

Next, as shown in Fig. 6 (Step q), the supernatant of liquid L5 in container 2d is removed with magnet 3c arranged in the vicinity of the outer wall at thebottomof container 2d. As a result, unreacted probe P2 contained in the supernatant of liquid L5 is removed, and the amount of unreacted probe P2 contained in the remainder of liquid L5 is reduced. When removing the supernatant of liquid L5, since hybrid C2 is maintained in an adhered state on the inner wall at the bottom of container 2d, a decrease in the amount of hybrid C2 is prevented.

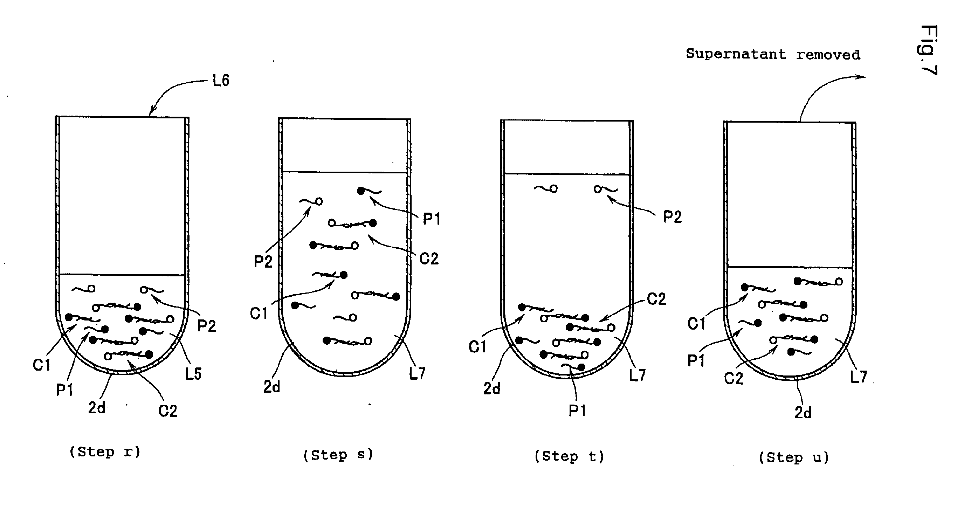

Next, as shown in Fig. 7 (Step r), a liquid L6 is injected into container 2d. This liquid L6 has a specific gravity which is greater than that of labeled non-magnetic particles LB, less than that of magnetic particles MB, and less than that of a particle complex composed oflabeled non-magnetic particlesLB and magnetic particles MB. At this time, the amount of liquid L5 in container 2d is adjusted so that the specific gravity of a liquid L7, obtained by mixing liquid L5 and liquid L6, is greater than that of labeled non-magnetic particles LB, less than that of magnetic particles MB, and less than that of a particle complex composed of magnetic particles MB and labeled non-magnetic particles LB.

As shown in Fig. 7 (Step s), liquid L7 is obtained in which is dispersed a unreacted probe P1, unreacted probe P2, hybrid C1 and hybrid C2.

Next, liquid L7 is allowed to stand undisturbed as shown in Fig. 7 (Step t).

Since the mass and volume of magnetic particles MB are much greater than the mass and volume of oligonucleotide N1, the mass and volume of labeled non-magnetic particles LB are much greater than the mass and volume of oligonucleotide N2, and the mass and volume of magnetic particles MB and labeled non-magnetic particles LB are much greater than the mass and volume of target nucleic acid T, the specific gravity of probe P1 approximates that of magnetic particles MB, the specific gravity of probe P2 approximates that of labeled non-magnetic particles LB, the specific gravity of hybrid C1 approximates that of magnetic particles LB, and the specific gravity of hybrid C2 approximates that of a particle complex composed of magnetic particles MB and labeled non-magnetic particles LB. Thus, when liquid L7 is allowed to stand undisturbed, although unreacted probe P1, hybrid C1 and hybrid C2 precipitate, unreacted probe P2 floats to the top. At this time, since forces other than gravity and buoyancy do not act on hybrid C2, destruction or degradation of hybrid C2 does not occur. In this manner, hybrid C2 is separated as a precipitate under mild conditions which do not cause destruction or degradation of hybrid C2.

Although the precipitate contains hybrid C2 along with unreacted probe P1 and hybrid C1, it does not contain unreacted probe P2. This is important when detecting hybrid C2 based on the label of labeled non-magnetic particles LB.

Next, as shown in Fig. 7 (Step s), the supernatant of liquid L7 is removed. As a result, the floating substance containing unreacted probe P2 is removed, and a precipitate containing unreacted probe P1, hybrid C1 and hybrid C2 remains in the remainder of liquid L7.

Next, hybrid C2 contained in the precipitate is detected based on the label of labeled non-magnetic particles LB.

In the case the label is a fluorescent label, labeled non-magnetic particles LB are made to luminesce by irradiating with an excitation light, and the amount of luminescence is measured with an optical measuring instrument such as a CCD camera, fluorescence scanner, spectrofluorometer or photomultiplier tube (PMT). In addition, in the case the label is a chemiluminescent label, the labeled non-magnetic particles LB are made to luminesce by supplying a luminescence trigger such as hydrogen peroxide, followed by measurement of the amount of luminescence with an optical measuring instrument such as PMT. In addition, in the case the label is an enzyme label, after adding a substrate solution and then supplying a reaction stopping solution, the solution is irradiated with a measuring beam of a predetermined wavelength to measure the absorbance of the solution.

In the present embodiment, two or more types of target nucleic acids can be detected in parallel. For example, in the case of detecting two types of target nucleic acids T and T' in parallel, probes P1 and P2 are used to detect target nucleic acid T, while probes P1' and P2' are used to detect target nucleic acid T'. Probe P1' is composed of magnetic particles MB' and oligonucleotide N1' immobilized on magnetic particles MB'. As shown in Fig. 11, oligonucleotide N1' is composed of a nucleotide sequence complementary to region R1' of target nucleic acid T', and is able to specifically hybridize with region R1' of target nucleic acid T'. In addition, probe P2' is composed of labeled non-magnetic particles LB' and oligonucleotide N2' immobilized on labeled non-magnetic particles LB'. As shown in Fig. 11, oligonucleotide N2' is composed of a nucleotide sequence complementary to region R2' of target nucleic acid T' , and is able to specifically hybridize with region R2' of target nucleic acid T'. The label of labeled non-magnetic particles LB and the label of labeled non-magnetic particles LB' can be detected separately. In the case of fluorescent labels, as a result of the types and amount ratios of the fluorescent compounds being different, the label of labeled non-magnetic particles LB and the label of labeled non-magnetic particles LB' can be detected separately.

In the present embodiment, particles having a specific gravity greater than that of magnetic particles MB can be selected for labeled non-magnetic particles LB, and a liquid having a specific gravity less than that of labeled non-magnetic particles LB, greater than that of magnetic particles MB, and greater than that of a particle complex composed of magnetic particles MB and labeled non-magnetic particles LB, can be selected for liquid L6. In this case, when liquid L7 is allowed to stand undisturbed, although unreacted probe P1, hybrid C1 and hybrid C2 float to the top, since unreacted probe P2 precipitates, a floating substance containing hybrid C2 is recovered, and hybrid C2 contained in the floating substance is detected based on the label of labeled non-magnetic particles LB.

In the present embodiment, although magnet 3a or 3b was moved closer to and away from container 2d with container 2d in a stationary state when magnet 3a or 3b was made to approach or move away from a predetermined region on the outer wall of container 2d, container 2d may be moved closer to or away from magnet 3a or 3b with magnet 3a or 3b in a stationary state. In addition, both container 2d and magnet 3a or 3b may be moved (for example, in the same direction or opposite directions).

In the present embodiment, although the location of the predetermined region of the outer wall of container 2d was changed from region F1 to region F2 and from region F2 to region F1, the change in the location of the predetermined region is not limited thereto. Since molecules containing magnetic particles MB can be moved in various directions if the location of the predetermined region is changed to various locations, the probability of probe P1, probe P2 and target nucleic acid T encountering each other can be improved, thereby making it possible to accelerate the reaction among probe P1, probe P2 and target nucleic acid T.

The location of the predetermined region of the outer wall of container 2d can be changed, for example, in the circumferential direction of container 2d, the lengthwise direction of container 2d, or a combined direction thereof.

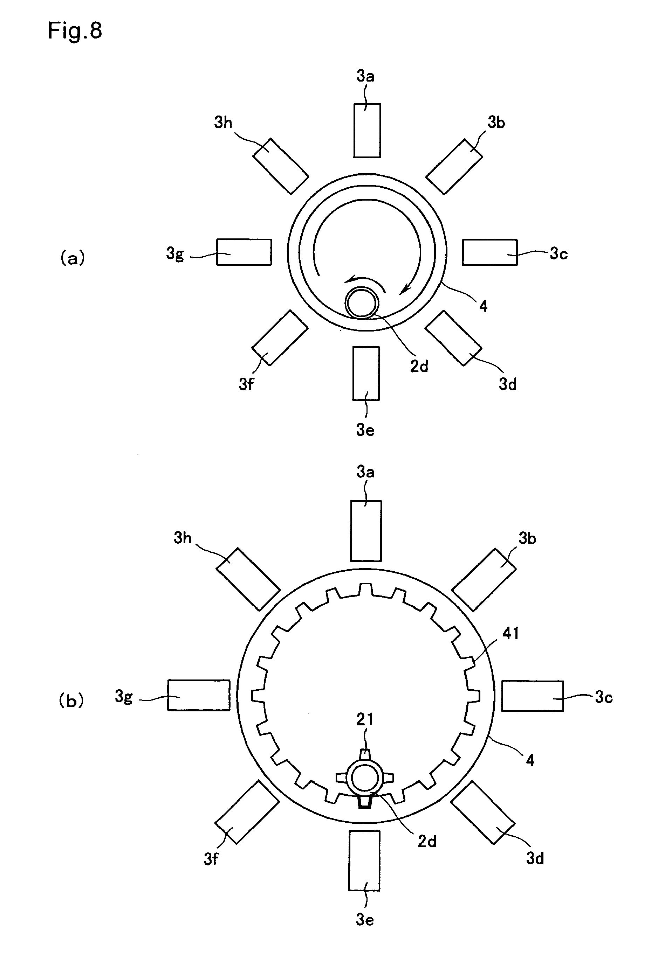

As shown in Fig. 8A, for example, by arranging a plurality of magnets 3a to 3h in the vicinity of the outer wall of a cylindrical member 4, and moving container 2d along the inner wall of cylindrical member 4 while rotating container 2d, each magnet is able to act on magnetic particles MB in container 2d through a predetermined region while changing the location of the predetermined region of the outer wall of container 2d in the circumferential direction of container 2d. At this time, as shown in Fig. 8B, by providing a plurality of protrusions 21 on the outer wall of container 2d, providing a plurality of indentations 41 in the inner wall of cylindrical member 4, and sequentially engaging protrusions 21 and indentations 41, container 2d can be moved along the inner wall of cylindrical member 4 while rotating container 2d. Furthermore, although 8 magnets are arranged in the vicinity of the outer wall of cylindrical member 4 in Figs. 8A and 8B, the number of magnets and locations at which they are arranged can be suitably altered.

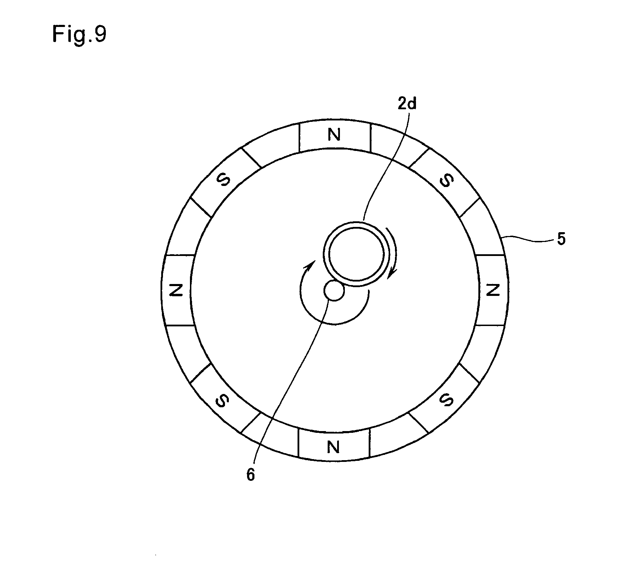

In addition, as shown in Fig. 9, by arranging a cylindrical member 6 in a through hole of a ring-shaped magnet 5 in which N poles and S poles have been suitably arranged, and moving container 2d along the outer wall of cylindrical member 6 while rotating container 2d, ring-shaped magnet 5 is able to act on magnetic particles in container 2d through each predetermined region while changing the location of the predetermined regions of the outer wall of container 2d in the circumferential direction of container 2d. At this time, similar to Fig. 8B, by providing a plurality of protrusions on the outer wall of container 2d, providing a plurality of indentations in the outer wall of cylindrical member 6, and sequentially engaging the protrusions and the indentations, container 2d can be moved along the outer wall of cylindrical member 6 while rotating container 2d.

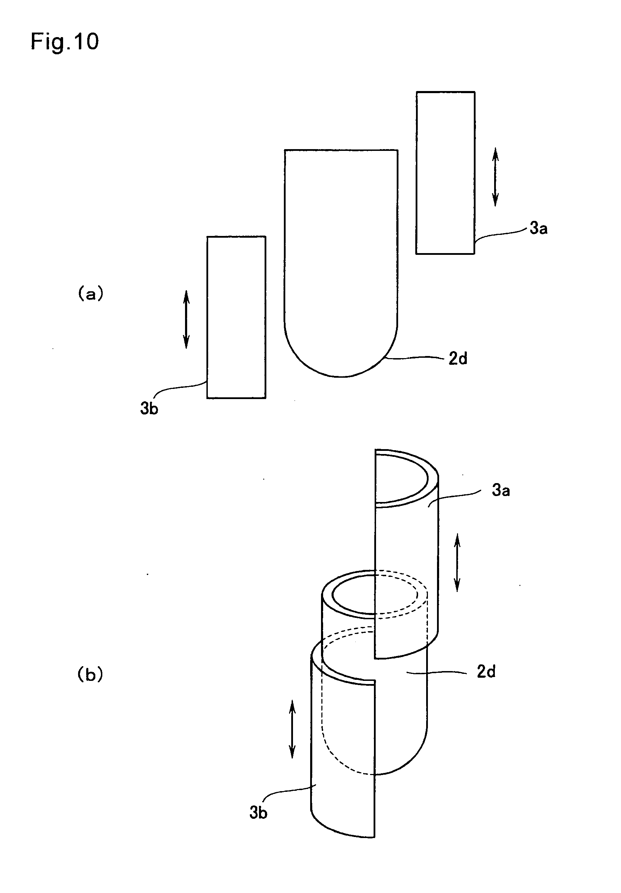

In addition, as shown in Figs. 10A and 10B, by respectively moving magnet 3a or 3b arranged in the vicinity of the outer wall of container 2d in the lengthwise direction of container 2d (vertical direction in Fig. 10), magnet 3a or 3b is able to act on magnetic particles in container 2d through each predetermined region while changing the location of the predetermined regions of the outer wall of container 2d in the lengthwise direction. The shape of magnet 3a or 3b can be that which follows the outer wall of container 2d as shown in Fig. 10B.

In the present embodiment, in the case magnet 3a or 3b is a temporary magnet such as an electromagnet, magnet 3a or 3b may be magnetized prior to approaching container 2d, or it may be magnetized after having approached container 2d. In addition, when canceling the action of the magnet, the action of the magnet may be canceled by demagnetizing the magnet after having approached container 2d.

In the present embodiment, although a bottomed cylindrical container was used for container 2d, a bottomless cylindrical container may also be used in the manner of pipette tip 10. A liquid can be retained in an aspirated state in the bottomless cylindrical container.

## Claims

1. A method for separating a complex of a first probe, a second probe and a target substance from a reaction mixture obtained by reacting the first probe having a first particle and a first binding substance immobilized on the first particle, the second probe having a second particle having a different specific gravity from the first particle and a second binding substance immobilized on the second particle, and the target substance having a first portion able to be bound by the first binding substance and a second portion able to be bound by the second binding substance, the method comprising:
a step in which, after mixing the reaction mixture with a liquid having a specific gravity greater than that of the first particle, less than that of the second particle and less than that of a particle complex composed of the first particle and the second particle, or a liquid having a specific gravity less than that of the first particle, greater than that of the second particle and greater than that of a particle complex composed of the first particle and the second particle, the mixture is allowed to stand undisturbed until a precipitate and a floating substance are formed.

2. The method according to claim 1, wherein the first particle and/or second particle is a magnetic particle, and
the reaction mixture is obtained by reacting the first probe, the second probe and the target substance in a reaction solvent housed in a container, followed by removing the reaction solvent in a state in which a magnet is arranged in the vicinity of the outer wall of the container.

3. The method according to claim 2, wherein the first particle is a non-magnetic particle, and the second particle is a magnetic particle.

4. A method for reacting a first probe having a first particle and a first binding substance immobilized on the first particle, a second probe having a second particle and a second binding substance immobilized on the second particle, and a target substance having a first portion able to be bound by the first binding substance and a second portion able to be bound by the second binding substance, wherein
the first particle and/or secondparticle is amagnetic particle, and the method comprises:
a first step in which the first probe, the second probe, the target substance and a reaction solvent are housed in a container,
a second step in which a magnet is allowed to act on the magnetic particle within the container through a predetermined region of the outer wall of the container,
a third step in which the action of the magnet is canceled, and
a fourth step in which the second and third steps are repeated while changing the location of the predetermined region.

5. The method according to claim 4, wherein the magnet is allowed to act by allowing the predetermined region and the magnet to approach each other in the second step,
the action of the magnet is canceled by separating the predetermined region from the magnet in the third step, and
the predetermined region and magnet are repeatedly allowed to approach and separate from each other while changing the location of the predetermined region in the fourth step.

6. The method according to claim 4 or claim 5, wherein the location of the predetermined region is changed in the circumferential direction or lengthwise direction of the container.

7. A method for detecting a target substance having a first portion able to be bound by a first binding substance and a second portion able to be bound by a second binding substance, using a first probe having a first particle and the first binding substance immobilized on the first particle, and a second probe having a second particle and the second binding substance immobilized on the second particle, the method comprising:
a first step in which a labeled particle is selected for the first particle and a particle having a specific gravity greater than that of the first particle is selected for the second particle,
a second step in which the first probe, the second probe and the target substance are reacted to obtain a reaction mixture,
a third step in which the reaction mixture is mixed with a liquid having a specific gravity greater than that of the first particle, less than that of the second particle, and less than that of a particle complex composed of the first particle and the second particle, followed by allowing to stand undisturbed until a precipitate and a floating substance are formed, and
a fourth step in which a complex of the first probe, the second probe and the target substance contained in the precipitate is detected based on the label of the first particle.

8. A method for detecting a target substance having a first portion able to be bound by a first binding substance and a second portion able to be bound by a second binding substance, using a first probe having a first particle and the first binding substance immobilized on the first particle, and a second probe having a second particle and the second binding substance immobilized on the second particle, the method comprising:
a first step in which a labeled particle is selected for the first particle and a particle having a specific gravity less than that of the first particle is selected for the second particle,
a second step in which the first probe, the second probe and the target substance are reacted to obtain a reaction mixture,
a third step in which the reaction mixture is mixed with a liquid having a specific gravity less than that of the first particle, greater than that of the second particle, and greater than that of a particle complex composed of the first particle and the second particle, followed by allowing to stand undisturbed until a precipitate and a floating substance are formed, and
a fourth step in which a complex of the first probe, the second probe and the target substance contained in the floating substance is detected based on the label of the first particle.

9. The method according to claim 7 or claim 8, wherein a magnetic particle is selected for the first particle and/or second particle in the first step, and
the reaction mixture is obtained by reacting the first probe, the second probe and the target substance in a reaction solvent housed in a container, followed by removing the reaction solvent in a state in which a magnet is arranged in the vicinity of the outer wall of the container in the second step.

10. The method according to claim 9, wherein a non-magnetic particle is selected for the first particle, and a magnetic particle is selected for the second particle in the first step.

11. The method according to claim 7 or claim 8, wherein a magnetic particle is selected for the first particle and/or second particle in the first step, and
the first probe, the second probe and the target substance are reacted by the method according to any of claims 4 to 6.

12. The method according to claim 11, wherein the reaction mixture is obtained by reacting the first probe, the second probe and the target substance, followed by removing the reaction solvent in the state in which a magnet is arranged in the vicinity of the outer wall of the container in the second step.

13. The method according to claim 12, wherein a non-magnetic particle is selected for the first particle, and a magnetic particle is selected for the second particle in the first step.

14. The method according to any of claims 7 to 13, wherein a magnetic particle is selected for the second particle in the first step, and
the first probe is reacted with a complex of the second probe and the target substance obtained by mixing a sample containing the target substance and the second probe, and recovering the complex of the second probe and the target substance by controlling magnetic force in the second step.
